# FASCICULE DE BREVET EUROPEEN

(11) **EP 2 385 058 B1**
(45) Date de publication et mention de la délivrance du brevet: **06.11.2013**
(21) Numéro de dépôt: 10183612.0
(22) Date de dépôt: 01.09.2000
(51) Int. Cl.: C07K 14/005, A61K 38/00, A61K 48/00

(54) **Procédé de détection de l'expression d'une protéine d'enveloppe d'un rétrovirus endogène humain et utilisations d'un gène codant pour cette protéine**
Verfahren zur Erkennung der Expression eines Hüllproteins eines endogenen humanen Retrovirus, und Anwendungen eines kodierenden Gens für dieses Protein
Method for detecting the expression of a human endogenous retrovirus envelope protein and uses of a coding gene for said protein

(30) Priorité: 01.09.1999 FR 9911141; 15.09.1999 FR 9911793
(43) Date de publication de la demande: 09.11.2011
(62) Demande divisionnaire de: 00960783.9
(73) Titulaire: BIO MERIEUX, 69280 Marcy l'Etoile (FR); INSERM - Institut National de la Santé et de la Recherche Médicale, 75013 Paris (FR)
(72) Inventeur: Mallet, François, 69100 Villeurbanne (FR); Cosset, François-Loïc, 69004 Lyon Cedex 07 (FR); Blond, Jean-Luc, 69290 Craponne (FR); Lavillette, Dimitri, 69007 Lyon (FR); Bouton, Olivier, 69110 Ste. Foy Les Lyon (FR); Ruggieri, Alessia, 69115 Heidelberg (FR)
(74) Mandataire: Maureau, Philippe

(56) Documents cités:
- WO-A-99/02696
- BLOND JEAN-LUC ET AL: "Molecular characterization and placental expression of HERV-W, a new human endogenous retrovirus family.", JOURNAL OF VIROLOGY, vol. 73, no. 2, février 1999 (1999-02), pages 1175-1185, XP000828006, ISSN: 0022-538X
- BLOND JEAN-LUC ET AL: "An envelope glycoprotein of the human endogenous retrovirus HERV-W is expressed in the human placenta and fuses cells expressing the type D mammalian retrovirus receptor.", JOURNAL OF VIROLOGY, vol. 74, no. 7, avril 2000 (2000-04), pages 3321-3329, XP000946327, ISSN: 0022-538X
- MI SHA ET AL: "Syncytin is a captive retroviral envelope protein involved in human placental morphogenesis.", NATURE (LONDON), vol. 403, no. 6771, 17 février 2000 (2000-02-17), pages 785-789, XP002147935, ISSN: 0028-0836

### LISTE DE SEQUENCES

<110> BIO MERIEUX
   INSTITUT NATIONAL DE LA SANTE ET DE LA RECHERCHE MEDICALE
<120> Procédé de détection de l'expression d'une protéine d'enveloppe d'un rétrovirus endogène humain et utilisations d'un gène codant pour cette protéine
<130> Pouvoir fusogène de env de ERV-W
<140>
   <141>
<160> 2
<170> PatentIn Ver. 2.1
<210> 1
   <211> 538
   <212> PRT
   <213> Homo sapiens
<400> 1
   Met Ala Leu Pro Tyr His Ile Phe Leu Phe Thr Val Leu Leu Pro Ser
    1               5                  10                  15
   Phe Thr Leu Thr Ala Pro Pro Pro Cys Arg Cys Met Thr Ser Ser Ser
               20                  25                  30
   Pro Tyr Gln Glu Phe Leu Trp Arg Met Gln Arg Pro Gly Asn Ile Asp
           35                  40                  45
   Ala Pro Ser Tyr Arg Ser Leu Ser Lys Gly Thr Pro Thr Phe Thr Ala
       50                  55                  60
   His Thr His Met Pro Arg Asn Cys Tyr His Ser Ala Thr Leu Cys Met
   65                  70                  75                  80
   His Ala Asn Thr His Tyr Trp Thr Gly Lys Met Ile Asn Pro Ser Cys
   85 90 95
   Pro Gly Gly Leu Gly Val Thr Val Cys Trp Thr Tyr Phe Thr Gln Thr
   100 105 110
   Gly Met Ser Asp Gly Gly Gly Val Gln Asp Gln Ala Arg Glu Lys His
   115 120 125
   Val Lys Glu Val Ile Ser Gln Leu Thr Arg Val His Gly Thr Ser Ser
   130 135 140
   Pro Tyr Lys Gly Leu Asp Leu Ser Lys Leu His Glu Thr Leu Arg Thr
   145 150 155 160
   His Thr Arg Leu Val Ser Leu Phe Asn Thr Thr Leu Thr Gly Leu His
   165 170 175
   Glu Val Ser Ala Gln Asn Pro Thr Asn Cys Trp Ile Cys Leu Pro Leu
   180 185 190
   Asn Phe Arg Pro Tyr Val Ser Ile Pro Val Pro Glu Gln Trp Asn Asn
   195 200 205
   Phe Ser Thr Glu Ile Asn Thr Thr Ser Val Leu Val Gly Pro Leu Val
   210 215 220
   Ser Asn Leu Glu Ile Thr His Thr Ser Asn Leu Thr Cys Val Lys Phe
   225 230 235 240
   Ser Asn Thr Thr Tyr Thr Thr Asn Ser Gln Cys Ile Arg Trp Val Thr
   245 250 255
   Pro Pro Thr Gln Ile Val Cys Leu Pro Ser Gly Ile Phe Phe Val Cys
   260 265 270
   Gly Thr Ser Ala Tyr Arg Cys Leu Asn Gly Ser Ser Glu Ser Met Cys
   275 280 285
   Phe Leu Ser Phe Leu Val Pro Pro Met Thr Ile Tyr Thr Glu Gln Asp
   290 295 300
   Leu Tyr Ser Tyr Val Ile Ser Lys Pro Arg Asn Lys Arg Val Pro Ile
   305 310 315 320
   Leu Pro Phe Val Ile Gly Ala Gly Val Leu Gly Ala Leu Gly Thr Gly
   325 330 335
   Ile Gly Gly Ile Thr Thr Ser Thr Gln Phe Tyr Tyr Lys Leu Ser Gln
   340 345 350
   Glu Leu Asn Gly Asp Met Glu Arg Val Ala Asp Ser Leu Val Thr Leu
   355 360 365
   Gln Asp Gln Leu Asn Ser Leu Ala Ala Val Val Leu Gln Asn Arg Arg
   370 375 380
   Ala Leu Asp Leu Leu Thr Ala Glu Arg Gly Gly Thr Cys Leu Phe Leu
   385 390 395 400
   Gly Glu Glu Cys Cys Tyr Tyr Val Asn Gln Ser Gly Ile Val Thr Glu
   405 410 415
   Lys Val Lys Glu Ile Arg Asp Arg Ile Gln Arg Arg Ala Glu Glu Leu
   420 425 430
   Arg Asn Thr Gly Pro Trp Gly Leu Leu Ser Gln Trp Met Pro Trp Ile
   435 440 445
   Leu Pro Phe Leu Gly Pro Leu Ala Ala Ile Ile Leu Leu Leu Leu Phe
   450 455 460
   Gly Pro Cys Ile Phe Asn Leu Leu Val Asn Phe Val Ser Ser Arg Ile
   465 470 475 480
   Glu Ala Val Lys Leu Gln Met Glu Pro Lys Met Gln Ser Lys Thr Lys
   485 490 495
   Ile Tyr Arg Arg Pro Leu Asp Arg Pro Ala Ser Pro Arg Ser Asp Val
               500                 505                 510
   Asn Asp Ile Lys Gly Thr Pro Pro Glu Glu Ile Ser Ala Ala Gln Pro
           515                 520                 525
   Leu Leu Arg Pro Asn Ser Ala Gly Ser Ser
       530                 535
<210> 2
   <211> 2781
   <212> ADN
   <213> Homo sapiens
<400> 2
   atgggagctg ttttcatgct atttcactct attaaatctt gcaactgcac tcttctggtc 60
   catgtttctt acggctcgag ctgagctttt gctcaccgtc caccactgct gtttgccacc 120
   accgcagacc tgccgctgac tcccatccct ctggatcctg cagggtgtcc gctgtgctcc 180
   tgatccagcg aggcgcccat tgccgctccc aattgggcta aaggcttgcc attgttcctg 240
   cacggctaag tgcctgggtt tgttctaatt gagctgaaca ctagtcactg ggttccatgg 300
   ttctcttctg tgacccacgg cttctaatag aactataaca cttaccacat ggcccaagat 360
   tccattcctt ggaatccgtg aggccaagaa ctccaggtca gagaatacga ggcttgccac 420
   catcttggaa gcggcctgct accatcttgg aagtggttca ccaccatctt gggagctctg 480
   tgagcaagga ccccccggta acattttggc aaccacgaac ggacatccaa agtgatacat 540
   cctgggaagg accctaccca gtcattttat ctaccccaac tgcggttaaa gtggctggag 600
   tggagtcttg gatacatcac acttgagtca aatcctggat actgccaaag gaacctgaaa 660
   atccaggaga caacgctagc tattcctgtg aacctctaga ggatttgcgc ctgctcttca 720
   aacaacaacc aggaggaaag taactaaaat cataaatccc catggccctc ccttatcata 780
   tttttctctt tactgttctt ttaccctctt tcactctcac tgcaccccct ccatgccgct 840
   gtatgaccag tagctcccct taccaagagt ttctatggag aatgcagcgt cccggaaata 900
   ttgatgcccc atcgtatagg agtctttcta agggaacccc caccttcact gcccacaccc 960
   atatgccccg caactgctat cactctgcca ctctttgcat gcatgcaaat actcattatt 1020
   ggacaggaaa aatgattaat cctagttgtc ctggaggact tggagtcact gtctgttgga 1080
   cttacttcac ccaaactggt atgtctgatg ggggtggagt tcaagatcag gcaagagaaa 1140
   aacatgtaaa agaagtaatc tcccaactca cccgggtaca tggcacctct agcccctaca 1200
   aaggactaga tctctcaaaa ctacatgaaa ccctccgtac ccatactcgc ctggtaagcc 1260
   tatttaatac caccctcact gggctccatg aggtctcggc ccaaaaccct actaactgtt 1320
   ggatatgcct ccccctgaac ttcaggccat atgtttcaat ccctgtacct gaacaatgga 1380
   acaacttcag cacagaaata aacaccactt ccgttttagt aggacctctt gtttccaatc 1440
   tggaaataac ccatacctca aacctcacct gtgtaaaatt tagcaatact acatacacaa 1500
   ccaactccca atgcatcagg tgggtaactc ctcccacaca aatagtctgc ctaccctcag 1560
   gaatattttt tgtctgtggt acctcagcct atcgttgttt gaatggctct tcagaatcta 1620
   tgtgcttcct ctcattctta gtgcccccta tgaccatcta cactgaacaa gatttataca 1680
   gttatgtcat atctaagccc cgcaacaaaa gagtacccat tcttcctttt gttataggag 1740
   cgggagtgct aggtgcacta ggtactggca ttggcggtat cacaacctct actcagttct 1800
   actacaaact atctcaagaa ctaaatgggg acatggaacg ggtcgccgac tccctggtca 1860
   ccttgcaaga tcagcttaac tccctagcag cagtagtcct tcaaaatcga agagctttag 1920
   acttgctaac cgctgaaaga gggggaacct gtttattttt aggggaagaa tgctgttatt 1980
   atgttaatca atccggaatc gtcactgaga aagttaaaga aattcgagat cgaatacaac 2040
   gtagagcaga ggagcttcga aacactggac cctggggcct cctcagccaa tggatgccct 2100
   ggattctccc cttcttagga cctctagcag ctataatatt gctactcctc tttggaccct 2160
   gtatctttaa cctccttgtt aactttgtct cttccagaat cgaagctgta aaactacaaa 2220
   tggagcccaa gatgcagtcc aagactaaga tctaccgcag acccctggac cggcctgcta 2280
   gcccacgatc tgatgttaat gacatcaaag gcacccctcc tgaggaaatc tcagctgcac 2340
   aacctctact acgccccaat tcagcaggaa gcagttagag cggtcgtcgg ccaacctccc 2400
   caacagcact taggttttcc tgttgagatg ggggactgag agacaggact agctggattt 2460
   cctaggctga ctaagaatcc ctaagcctag ctgggaaggt gaccacatcc acctttaaac 2520
   acggggcttg caacttagct cacacctgac caatcagaga gctcactaaa atgctaatta 2580
   ggcaaagaca ggaggtaaag aaatagccaa tcatctattg cctgagagca cagcaggagg 2640
   gacaatgatc gggatataaa cccaagtctt cgagccggca acggcaaccc cctttgggtc 2700
   ccctcccttt gtatgggagc tctgttttca tgctatttca ctctattaaa tcttgcaact 2760
   gcaaaaaaaa aaaaaaaaaa a                                           2781

## Description

Les rétrovirus sont des virus enveloppés qui portent des spicules glycoprotéiques codées par les virus à leur surface. Ces glycoprotéines d'enveloppe sont synthétisées sous la forme de précurseurs polyprotéiques (Pré-env) qui sont ensuite clivés par des protéases cellulaires en protéine de surface mature (SU) et en protéine transmembranaire (TM). Les glycoprotéines d'enveloppe sont impliquées dans l'entrée des virus dans les cellules hôte. Elles reconnaissent spécifiquement et se lient à des récepteurs de surface cellulaire et sont nécessaires pour la fusion de l'enveloppe virale et des membranes cellulaires de l'hôte. Le récepteur et l'enveloppe sont des molécules multamériques ou oligomériques. Pour tous les virus enveloppés, les interactions des glycoprotéines d'enveloppe avec le ou les récepteur(s) cellulaire(s) conduisent à des réarrangements conformationnels de l'enveloppe nécessaires à l'exposition du peptide de fusion. La fusion a lieu à la surface de la cellule ou dans des vésicules cellulaires suivant la voie d'endocytose du virion. De plus, pour permettre l'entrée du virus, une fusion médiée par les protéines de surface virales peut, dans certaines conditions, provoquer une fusion cellule à cellule avec pour résultat la formation de cellules multinucléées géantes ou syncytia. La formation de syncytia est réalisée par au moins deux voies : un virion peut simultanément fusionner avec deux cellules, on parle alors de fusion " from without ", ou une cellule infectée qui exprime les glycoprotéines d'enveloppe à sa surface peut fusionner avec une cellule adjacente (fusion " from within ").

Les déterminants de l'enveloppe et la séquence des événements causant les changements conformationnels de l'enveloppe lors des processus de fusion " from without" sont bien documentés pour les orthomyxovirus qui nécessitent un environnement acide des vésicules d'endocytose pour leur entrée (Skehel, J. J. et al., PNAS, 79 :968-972 (1982)). Pour les rétrovirus, pour lesquels la voie d'entrée est indépendante du pH, les déterminants précis et les étapes, menant de la reconnaissance du récepteur à l'activation de la fusion ne sont pas encore élucidés. D'autres rétrovirus sont connus pour induire une fusion cellule à cellule (" fusion from within "), tels que le virus de la leucémie féline, le virus de la tumeur mammaire de la souris, le virus de la réticuloendothéliose aviaire, VIH et SIV.

Par ailleurs, Fefferey S. Jones et Rex Risser (Journal of Virology, Janv. 1993, p 67-74) ont montré que les glycoprotéines d'enveloppe du virus de la leucémie murine écotrope (MuLV) de type sauvage, sous la dépendance du LTR viral, étaient capables d'induire la formation de syncytia dans des cellules de rat XC en l'absence de virion (fusion " from within ").

De la connaissance des inventeurs, il n'a jamais été montré de pouvoir fusogène, dans un processus de fusion " from within ", des glycoprotéines d'enveloppe d'un rétrovirus endogène humain.

Des auteurs ont bien émis l'hypothèse que l'enveloppe rétrovirale endogène de ERV3, un rétrovirus endogène humain proche de MLV (Moloney Leukemia Virus), pouvait être impliquée *in vivo* dans l'élaboration du placenta via un processus de fusion (Patrick J. W. Venables et al., Virology, 211, 589-592 (1995)) mais ce phénomène n'a jamais été démontré *in vitro.* D'autre part, les études sur le polymorphisme de *env* ERV3, sur des individus d'origine caucasienne, ont permis de mettre en évidence la présence d'une mutation dans la région (SU) de l'enveloppe ERV3 générant un codon stop précoce présent à l'état homozygote dans 1% de la population étudiée, sans que ces individus présentent d'anomalie de la grossesse ou du développement placentaire (Nathalie de Parseval et Thierry Heidmann, Journal of Virology, Vol ; 72, N° 4, pages 3442-3445 (1998)), mettant ainsi en cause l'hypothèse précédemment émise.

Les présents inventeurs ont maintenant mis en évidence *in vitro* que la glycoprotéine d'enveloppe de HERV-W non modifiée, exprimée sous la dépendance d'un promoteur, de préférence un promoteur hétérologue, possède des propriétés fusogènes.

HERV-W est une famille de rétrovirus endogènes humains multi-copie récemment décrite, dénommée ainsi en raison de l'homologie entre le site de fixation de l'amorce de la transcription inverse et celui des rétrovirus aviaires utilisant l'ARNt Trp. Aucune entité compétente pour sa réplication n'a été mise en évidence. La fonctionnalité d'une région promotrice a été vérifiée et, parmi différents tissus humains sains testés, son expression semble être restreinte au placenta par Northern Blot (J. L Blond et al., Journal of Virology, Vol. 73, N° 2, pages 1175-1185 (1999)). Un cadre ouvert de lecture unique codant pour une enveloppe rétrovirale potentiellement fonctionnelle existe sur le chromosome 7. Un clone ADNc correspondant vraisemblablement à un transcrit sous génomique et portant la séquence de l'enveloppe complète a été isolé à partir de matériel placentaire (clone cl.PH74, GenBank AF072506, dont la séquence est identifiée par SEQ ID NO : 2). Les études phylogénétiques effectuées au niveau protéique indiquent que la protéine d'enveloppe est de type D. La séquence SEQ ID NO : 2 donnée en fin de description correspond donc à la séquence nucléotidique en ADNc complète du clone cl.PH74 dont la séquence protéique est identifiée par SEQ ID NO :1.

Env HERV-W possède tous les " attributs " d'une enveloppe rétrovirale : en particulier, un peptide leader, les deux sous unités caractéristiques SU et TM séparées par un site de clivage par les furines et, au niveau de sa TM, elle possède un peptide de fusion hydrophobe, une région immunosuppressive et une région carboxyl transmembranaire suivie d'une queue cytoplasmique longue. L'expression de Env HERV-W a été mise en évidence dans le placenta.

Les expériences réalisées par les inventeurs montrent que Env HERV-W entraîne, par fusion de cellule à cellule, la formation de syncytia dans différentes lignées cellulaires testées d'origine humaine et simienne. Le phénomène de fusion observé est dépendant de la reconnaissance de récepteur(s) spécifique(s), comme montré de manière directe lors de transfections et de manière indirecte lors de co-cultures des cellules transfectées avec d'autres types cellulaires. Les présents inventeurs ont par ailleurs identifié le récepteur spécifique de Env HERV-W par une approche de compétition s'appuyant sur la propriété d'interférence des enveloppes rétrovirales, en bloquant des récepteurs cellulaires par une protéine d'enveloppe autre que Env HERV-W, empêchant ainsi la formation de syncytia. Le récepteur identifié par les présents inventeurs est le récepteur hATB° des rétrovirus mammifères de type D exprimé dans les cellules humaines (Rasko E. J. et al. PNAS, 1999, 96 : 2129-2134 et Tailor C. S. et al. J. Virol., 1999, 73(5) : 4470-4474).

Les inventeurs ont mis au point un procédé de détection de l'expression d'une protéine ou d'un polypeptide d'enveloppe d'un rétrovirus endogène humain, selon lequel la protéine ou le polypeptide présente une séquence polypeptidique qui comprend la séquence SEQ ID NO :1 ou un fragment de SEQ ID NO :1, ou une séquence présentant, pour toute suite de 20 acides aminés, au moins 80 %, de préférence au moins 90% ou encore au moins 95% d'identité avec la séquence SEQ ID NO:1 ou avec un fragment de SEQ ID NO :1, et selon lequel on détecte le pouvoir fusogène de ladite protéine ou dudit fragment dans des cellules d'un tissu cellulaire ou d'une culture cellulaire, par la mise en évidence de la formation de syncytia.

Ils ont aussi mis au point un procédé de détection de l'expression d'une protéine ou d'un polypeptide d'enveloppe d'un rétrovirus endogène humain, selon lequel la protéine ou le polypeptide présente une séquence polypeptidique présentant, pour toute suite de 20 acides aminés, au moins 80 %, de préférence au moins 90% ou encore au moins 95% d'identité avec la séquence SEQ ID NO:1, et selon lequel on détecte le pouvoir fusogène de ladite protéine dans des cellules d'un tissu cellulaire, ou d'une culture cellulaire par la mise en évidence de la formation de syncytia.

Ladite protéine ou ledit polypeptide présente une séquence polypeptidique qui comprend la séquence SEQ ID NO :1 ou un fragment de SEQ ID NO :1, ou une séquence présentant, pour toute suite de 20 acides aminés, au moins 80%, de préférence au moins 90%, ou encore au moins 95%, d'identité avec la séquence SEQ ID NO:1 ou avec un fragment de SEQ ID NO :1.

Il est bien entendu que ladite protéine ou ledit polypeptide, ou leurs dits fragments, s'ils ne présentent pas une identité complète avec SEQ ID NO :1 ou ses fragments, doivent posséder un pouvoir fusogène, de préférence au moins égal ou supérieur à celui de SEQ ID NO :1 ou ses fragments.

Si les fragments de la protéine ou du polypeptide ci-dessus présentent une identité complète avec les fragments de SEQ ID NO :1, alors la taille de ces fragments peut être inférieure à 20 acides aminés, par exemple elle peut être d'environ 10 acides aminés, voire d'environ 5 acides aminés.

Les variations prévues dans la séquence polypeptidique de la protéine ou du polypeptide ou de leurs fragments comprennent les variations liées au polymorphisme, mais aussi les modifications telles que substitution(s), délétion(s) et addition(s) susceptibles d'être apportées à ladite séquence polypeptidique pour obtenir une protéine, un polypeptide ou un fragment de ceux-ci possédant un pouvoir fusogène, en particulier au moins égal ou supérieur à celui de SEQ ID NO :1 ou ses fragments.

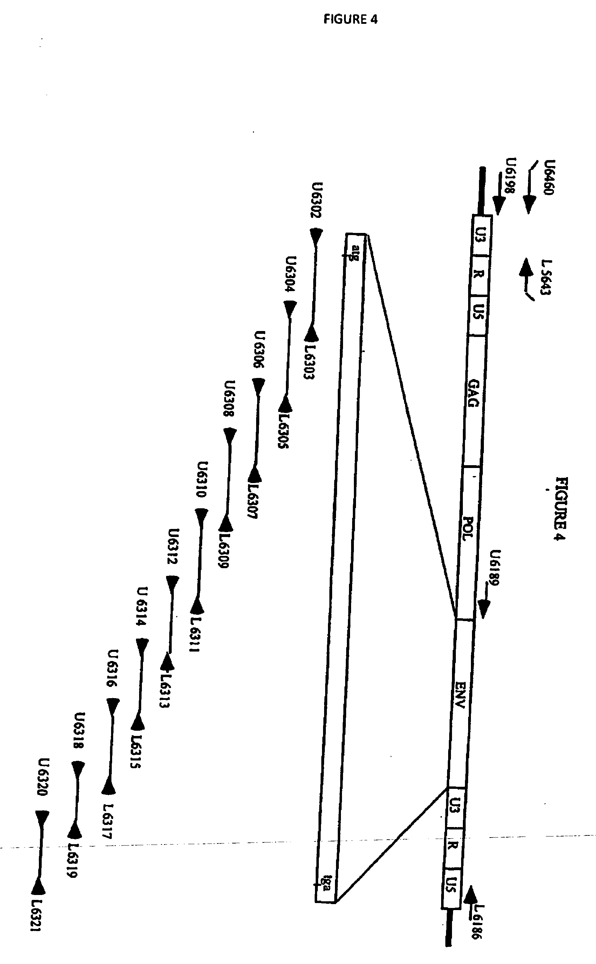

L'analyse du polymorphisme peut être réalisée par la méthode SSCP (Single Strand Conformational Polymorphism), qui est une méthode électrophorétique permettant d'objectiver, à l'aide de différences de migration, la présence d'au moins une mutation discriminant deux séquences courtes (inférieures à 250 pb). Ainsi, comme illustré à la figure 4, après amplification sur ADN total à l'aide des amorces U6198 et L6186 ou U 6189et L6186, il est possible d'analyser le polymorphisme de l'enveloppe localisée sur le chromosome 7 à l'aide du jeu d'amorces représenté (U6302 à L6321), permettant de générer un ensemble de 10 fragments chevauchants de taille adéquate. Le polymorphisme de l'un des sous-fragments peut également être mis en évidence par des techniques de séquençage, de cartographie de restriction le cas échéant, ou plus simplement par une technique d'hybridation sandwich de type ELOSA permettant de discriminer jusqu'à une mutation ponctuelle (Cros P. et al., demande de brevet européen EP 0 486 661).

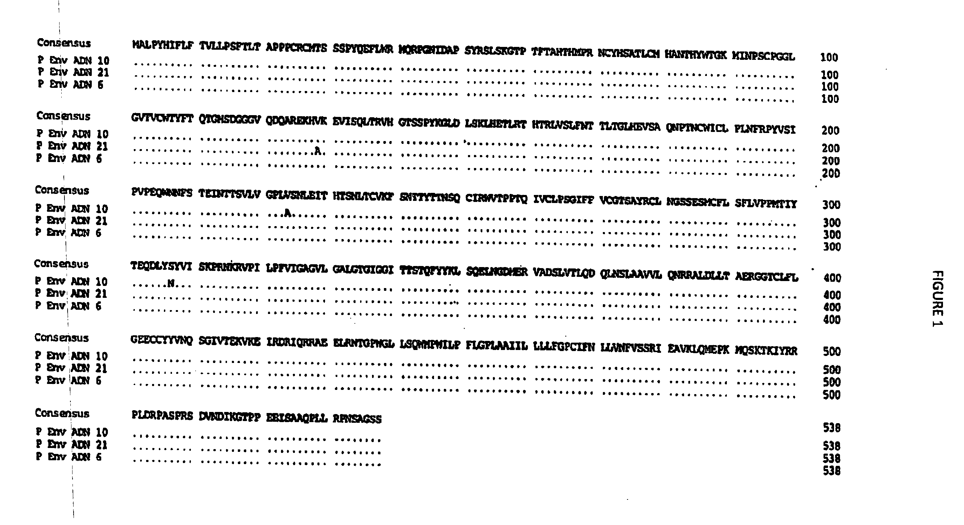

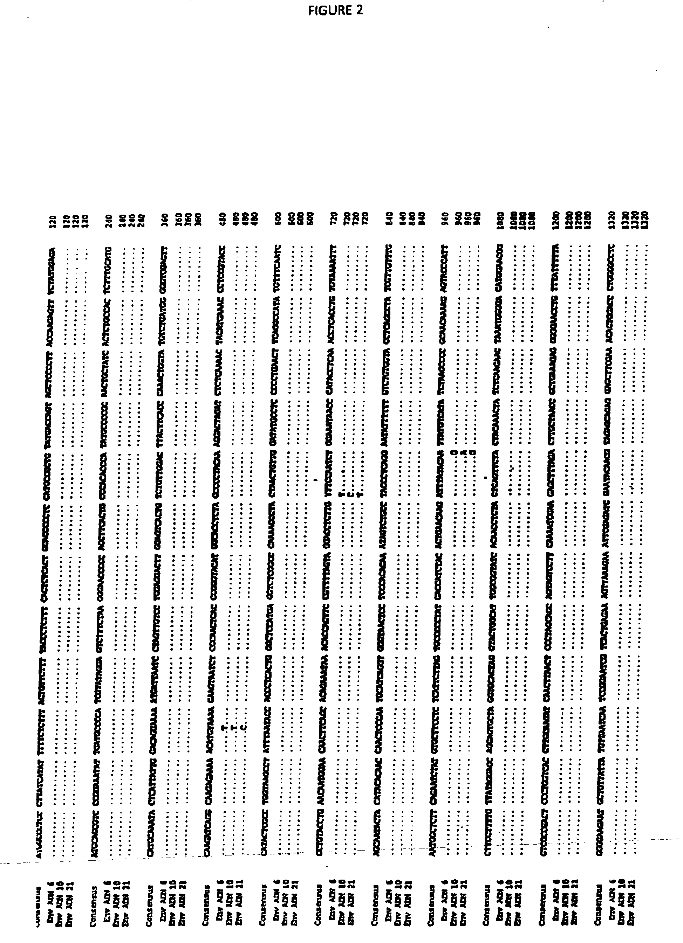

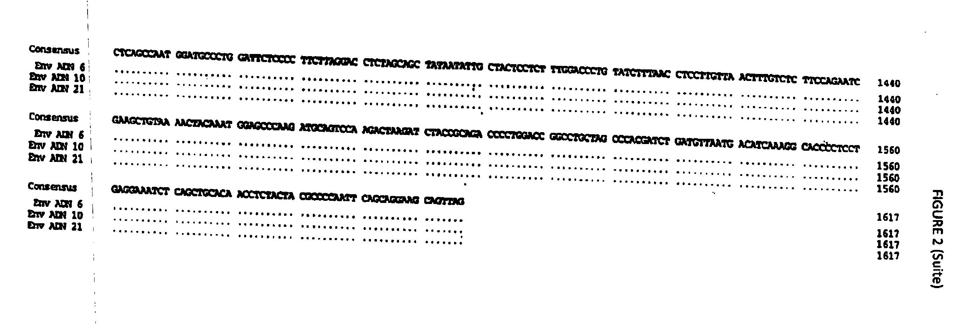

Des exemples de séquences Env HERV-W polymorphes sont représentés à la figure 1 annexée, les séquences ADN correspondantes étant représentées à la figure 2. Ces figures représentent l'alignement de séquences protéiques et nucléiques obtenues par séquençage de clones issus de trois individus différents.

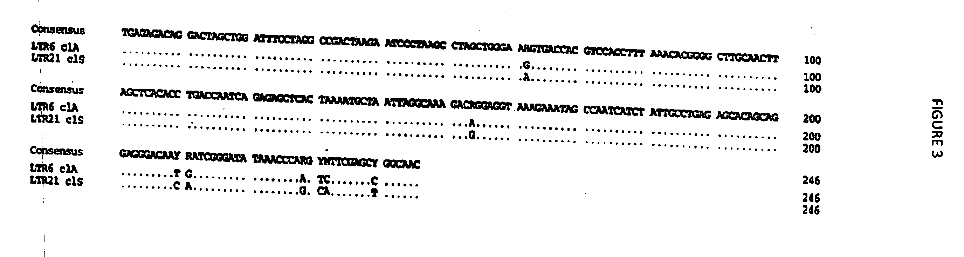

Par ailleurs, le polymorphisme du LTR qui dirige la transcription du gène *env* situé sur le chromosome 7 a été étudié. On observe deux groupes de LTRs 5' dont les séquences nucléiques obtenues par séquençage de deux clones provenant de deux individus différents sont représentées et alignées dans la figure 3.

Un choix judicieux d'amorces a permis d'amplifier spécifiquement sur le chromosome 7, à partir d'ADN humain total, un fragment nucléique contenant l'intégralité de l'information U3RU5-gag-pol-env-U3RU5 à l'aide des amorces U6198, L6186 ou exclusivement la séquence env-U3RU5 à l'aide des amorces U6189, L6186. Une telle approche est par exemple possible en utilisant une amorce chevauchant la zone de jonction entre la séquence rétrovirale (U3 en amont, U5 en aval) et la séquence flanquante non rétrovirale contiguë. Par, exemple, l'amorce L6186 chevauche la région U5 3' terminale et la séquence non rétrovirale avale. A partir d'un tel produit de PCR isolant la séquence d'intérêt du mélange des séquences HERV-W présentes dans le génome humain, il est possible de réaliser une analyse du polymorphisme.

De manière préférentielle, ladite protéine présente au moins l'une des caractéristiques suivantes :
- elle est codée par le gène *env* du rétrovirus endogène HERV-W ;
- elle est codée par un cadre de lecture ouvert situé sur le chromosome 7 du génome humain ;
- elle présente une séquence polypeptidique qui comprend la séquence SEQ ID NO : 1, ou une séquence présentant pour toute suite de 20 acides aminés, au moins 80%, de préférence au moins 90%, ou encore au moins 95% d'identité avec la SEQ ID NO :1. De préférence, elle consiste en SEQ ID NO:1.

De manière préférentielle, le polypeptide présente une séquence polypeptidique qui commence à l'acide aminé 448 et se termine à l'acide aminé 538 de SEQ ID NO : 1 ou une séquence polypeptidique présentant, pour toute suite de 20 acides aminés, au moins 80%, de préférence au moins 90%, ou encore au moins 95% d'identité avec la séquence polypeptidique qui commence à l'acide aminé 448 et se termine à l'acide aminé 538 de SEQ ID NO:1. De préférence, il consiste en une séquence polypeptidique qui commence à l'acide aminé 448 et se termine à l'acide aminé 538 de SEQ ID NO:1. Le polypeptide qui répond à la définition ci-dessus est un élément de régulation pouvant conférer ou restituer sa capacité fusogénique à une enveloppe rétrovirale non réputée fusogène dans un test de fusion cellule-cellule.

Les cellules dudit tissu ou de ladite culture cellulaire dans lesquelles on recherche à mettre en évidence le pouvoir fusogène sont avantageusement choisies parmi les cellules osseuses, les cellules musculaires, les cellules placentaires, les cellules endothéliales, en particulier des vaissaux sanguins, les cellules épithéliales, les cellules gliales et les cellules tumorales ou issues de lignées cellulaires tumorales.

Comme cela sera illustré dans les exemples qui suivent, la détection du pouvoir fusogène de ladite protéine ou dudit polypeptide peut être mise en oeuvre selon au moins l'un quelconque des deux protocoles suivants.

Selon un premier protocole, on obtient un vecteur d'expression de ladite protéine ou dudit polypeptide à partir duquel l'expression de la protéine, du polypeptide ou de son gène est sous la dépendance d'un promoteur, de préférence un promoteur fort ; on transfecte des cellules par le vecteur obtenu pour obtenir des cellules productrices, exprimant à leur surface ladite protéine ou ledit polypeptide; et on observe la formation de syncytia ou l'absence de formation de syncytia.

Selon un second protocole, on obtient un vecteur d'expression de ladite protéine ou dudit polypeptide à partir duquel l'expression de la protéine, du polypeptide ou de son gène est sous la dépendance d'un promoteur, de préférence un promoteur fort; on transfecte des cellules par le vecteur obtenu pour obtenir des cellules productices, exprimant à leur surface ladite protéine ou ledit polypeptide; on co-cultive des cellules naïves indicatrices exprimant à leur surface un récepteur de ladite protéine en présence desdites cellules productices ; et on observe la formation de syncytia ou l'absence de formation de syncytia.

Un gène ou un acide nucléique ou un fragment de gène ou un acide nucléique codant pour une protéine ou un polypeptide tel que défini précédemment dans la description peut être utilisé dans des conditions appropriées permettant son expression, pour préparer une composition thérapeutique ou prophylactique.

Une telle composition peut comprendre en outre un promoteur hétérologue ou autologue, de préférence hétérologue, pour l'expression de ladite protéine ou dudit polypeptide.

D'autres applications sont les suivantes :
- un vecteur d'expression comprenant au moins un gène ou un acide nucléique ou un fragment de gène ou d'acide nucléique codant pour une protéine ou un polypeptide tels que définis précédemment, et des éléments nécessaires à son expression dans une cellule hôte ;
- une cellule hôte comprenant au moins un vecteur d'expression ci-dessus , et
- une composition thérapeutique ou prophylactique comprenant au moins un vecteur d'expression ou une cellule hôte ci-dessus.

Les différentes compositions thérapeutiques décrites précédemment sont en particulier destinées au traitement de cancers, tel que par destruction des cellules cancéreuses au moyen de la formation de syncytia. Les différentes compositions prophylactiques décrites précédemment sont notamment destinées à prévenir une déficience dans l'élaboration du placenta.

Les compositions thérapeutiques ou prophylactiques, telles que définies ci-dessus, sont avantageusement destinées à un traitement communément dénommé « traitement par thérapie génique » ou « traitement par transfert de gène ».

Comme énoncé ci-dessus, les propriétés fusogènes de la protéine Env HERV-W, du polypeptide Env HERV-W ou de leurs fragments tels que définis ci-dessus trouvent notamment une application dans le domaine de la thérapie génique des cancers.

Ainsi, un objet de l'invention est une utilisation d'un fragment d'acide nucléique codant pour l'expression d'un peptide d'une protéine d'enveloppe du rétrovirus endogène humain HERV-W, ledit peptide commençant à l'acide aminé 448 et se terminant à l'acide aminé 538 de SEQ ID NO : 1, pour préparer une composition de thérapie génique, destinée au traitement de cancers par destruction des cellules indicatrices cancéreuses exprimant le récepteur hATB°, au moyen de la formation de syncytia.

Un autre objet de l'invention est une utilisation d'un gène codant pour l'expression d'une protéine d'enveloppe du rétrovirus endogène humain HERV-W, ladite protéine comprenant ou consistant en SEQ ID NO: 1, pour préparer une composition de thérapie génique, destinée au traitement de cancers par destruction des cellules indicatrices cancéreuses exprimant le récepteur hATB°, au moyen de la formation de syncytia.

A ce jour les gènes les plus fréquemment utilisés dans la thérapie contre les cancers sont (i) les gènes qui codent pour des protéines qui augmentent l'immunogénicité des cellules tumorales, telles que les cytokines pro-inflammatoires, (ii) les gènes qui codent pour des enzymes qui rendent les cellules cancéreuses sensibles à un pro-médicament dans des systèmes gène/pro-drogue, tels que le système thymidine kinase du virus Herpes Simplex/Ganciclovir ou le système cytosine désaminase/5FC.

De manière idéale, le transfert de gènes thérapeutiques devrait conduire à la fois à une destruction locale des cellules cancéreuses, à l'activation de l'immunité anti-tumorale pour éliminer les zones tumorales auxquelles les gènes thérapeutiques ne peuvent être délivrés et le traitement ne devrait pas causer de dommages aux tissus cellulaires normaux de l'hôte, en particulier aux tissus des organes vitaux.

La protéine ou le polypeptide qui comprend ou consiste en la protéine Env HERV-W ou ses fragments, en particulier un fragment qui commence à l'acide aminé 448 et se termine à l'acide aminé 538 de SEQ ID NO : 1, ou en une séquence polypeptidique présentant pour toute suite de 20 acides aminés, au moins 80%, de préférence au moins 90% ou encore au moins 95% d'identité avec SEQ ID NO :1 ou un fragment de SEQ ID NO : 1 et en particulier le fragment identifié ci-dessus, sous la dépendance d'un promoteur hétérologue ou autologue capable d'induire son expression répond aux critères définis ci dessus via la formation de syncytia. Ces syncytia se forment à partir de cellule(s) transfectée(s) par un processus de fusion de cellule à cellule.

Dans un mode de réalisation en vu d'optimiser ses caractéristiques thérapeutiques, la protéine ou le polypeptide ci-dessus ou tout fragment est éventuellement fusionné avec d'autres protéine(s) ou fragment(s) de protéine(s), même si intrinsèquement il répond aux critères définis précédemment. A contrario, tout ou partie de la protéine, et en particulier le polypeptide dont la séquence peptidique comprend ou consiste en la séquence qui commence à l'acide aminé 448 et se termine à l'acide aminé 538 de SEQ ID NO : 1, peut être fusionné à d'autres protéines en vue de leur conférer des propriétés particulières. La protéine ou le polypeptide ci-dessus ou son fragment est capable d'induire la formation de syncytia à un pH voisin de la neutralité ou à pH neutre. Typiquement le vecteur d'expression ou plasmide sera adapté pour permettre l'expression de la protéine, du polypeptide ou fragment induisant la formation de syncytia, de telle sorte que lorsqu'il est exprimé la protéine, le polypeptide ou fragment puisse induire la fusion des cellules transfectées avec d'autres cellules humaines non transfectées. Il est souhaitable que la protéine ou le polypeptide soit exprimé indépendamment d'autres composants viraux, à moins que ceux ci soient utiles à la vectorisation.

Aussi, un gène ou un acide nucléique, ou un fragment de gène ou d'un acide nucléique, recombinant codant pour une protéine ou un polypeptide ou un fragment ci-dessus qui induit la formation de syncytia par fusion de cellules transformées et de cellules malignes cibles peut être utilisé dans le domaine de la thérapie de maladies malignes, telles que les cancers.

Une méthode de traitement d'une maladie maligne chez un patient peut consister à administrer au patient le gène ou un acide nucléique, ou un fragment de gène ou d'un acide nucléique, recombinant codant pour une protéine ou un polypeptide ou un fragment ci-dessus qui induit la formation de syncytia par fusion de cellules transformées et de cellules malignes cibles.

Le gène ou l'acide nucléique, ou le fragment de gène ou d'acide nucléique est introduit *in vitro* dans des cellules humaines appropriées, telles que des cellules de lignées continues immortalisées, par des techniques standards connues de l'homme du métier, telle que transfection, transduction ou transformation, et les cellules ainsi transformées sont ensuite introduites chez le patient où elles peuvent exercer leurs propriétés fusogènes.

Le gène ou l'acide nucléique, ou le fragment de gène ou d'acide nucléique ci-dessus peut être utilisé de différentes manières pour le traitement de cancers, en particulier pour le traitement de tumeurs solides ou molles. Les cellules cibles peuvent être transformées *ex vivo* ou *in vivo* par les vecteurs (plasmides) codant pour le polypeptide ci-dessus.

Les propriétés fusogènes de la protéine Env HERV-W, du polypeptide Env HERV-W ou de leurs fragments tels que définis dans la présente description trouvent également une application dans le domaine de la prophylaxie pour prévenir une déficience dans l'élaboration du placenta et pallier des échecs de grossesse.

Le gène ou l'acide nucléique ou leurs fragments tels que définis ci-dessus peuvent donc être utilisés pour différents effets thérapeutiques ou prophylactiques, le but ultime étant soit (i) de détruire les cellules cibles par formation de syncytia induisant une mort cellulaire des cellules cibles par un processus de mort différent de la mort cellulaire par apoptose, soit (ii) d'induire ou de favoriser la formation de syncytia, par exemple pour pallier une déficience dans la formation des syncytiotrophoblastes lors de la grossesse, ou pour prévenir une déficience dans la formation naturelle de tout autre type de syncytia, ladite déficience étant associée à une pathologie.

La protéine Env HERV-W ou d'un fragment de Env HERV-W, tels que définis précédemment, peuvent être utilisés à la surface d'un vecteur de thérapie génique comprenant, entre autres, un gène ou une séquence d'acide nucléique ou un oligonucléotide d'intérêt thérapeutique susceptible d'être exprimé dans une cellule cible ou de s'hybrider à une séquence nucléotidique complémentaire d'une cellule cible, ladite protéine Env HERV-W ou ledit fragment de cette protéine interagissant avec son récepteur cellulaire décrit ci-dessus, favorisant ainsi l'introduction du gène ou de la séquence d'acide nucléique ou de l'oligonucléotide d'intérêt thérapeutique dans la cellule cible.

Un vecteur de thérapie génique peut comprendre une protéine ou un polypeptide ou un fragment d'enveloppe d'un rétrovirus endogène humain, ladite protéine ou ledit polypeptide présentant une séquence polypeptidique qui comprend la séquence SEQ ID NO : 1 ou un fragment de SEQ ID NO :1, en particulier un fragment dont la séquence peptidique comprend ou consiste en la séquence qui commence à l'acide aminé 448 et se termine à l'acide aminé 538 de SEQ ID NO : 1, ou une séquence présentant pour toute suite de 20 acides aminés, au moins 80%, de préférence au moins 90% ou encore au moins 95% d'identité avec la séquence SEQ ID NO :1 ou avec un fragment de SEQ ID NO : 1, en particulier tel que défini ci-dessus. De préférence, ce vecteur de thérapie génique comprend la séquence SEQ ID NO :1. Dans un mode de réalisation particulier, le vecteur de thérapie génique précédemment cité consiste en un vecteur rétroviral classique de type MLV ou en un vecteur lentiviral pseusotypés par tout ou partie de la protéine d'enveloppe de HERV-W telle que définie ci-dessus, ou encore en un vecteur synthétique portant à sa surface tout ou partie de la protéine Env HERV-W telle que définie ci-dessus conférant les propriétés de ciblage cellulaire et de fusion de la membrane plasmatique.

Un vecteur de thérapie génique peut comprendre à sa surface le récepteur de la protéine identifiée en SEQ ID NO :1, notamment pour cibler des cellules produisant la protéine identifiée en SEQ ID NO :1 de façon constitutive ou induite.

Les séquences d'acides nucléiques et/ou oligonucléotides d'intérêt thérapeutique (anti-sens ou codant pour une protéine) permettent notamment de cibler les cellules dans lesquelles un gène est exprimé.

Les séquences d'acides nucléiques ou les oligonucléotides anti-sens sont capables d'interférer spécifiquement avec la synthèse d'une protéine cible, par inhibition de la formation et/ou du fonctionnement du polysome, selon le positionnement de l'anti-sens dans l'ARNm de la cible. Donc, le choix fréquent de la séquence entourant le codon d'initiation de la traduction comme cible pour une inhibition par une séquence d'acide nueléique anti-sens ou par un oligonuclétide antisens vise à prévenir la formation du complexe d'initiation. D'autres mécanismes dans l'inhibition par des oligonucléotides anti-sens impliquent une activation de la ribonucléase H qui digère les hybrides oligonucléotide anti-sens/ARNm ou une interférence au niveau de sites d'épissage par des oligonucléotides anti-sens dont la cible est un site d'épissage de l'ARNm. Les oligonucléotides anti-sens sont également complémentaires de séquences ADN et peuvent donc interférer au niveau de la transcription par la formation d'une triple hélice, l'oligonucléotide anti-sens s'appariant par des liaisons hydrogène dites de Hoogsteen au niveau du grand sillon de la double hélice d'ADN. Dans ce cas particulier, on parle plus précisément d'oligonucléotides antigènes. Il est bien entendu que les séquences d'acides nucléiques ou oligonucléotides anti-sens peuvent être strictement complémentaires de la cible ADN ou ARN à laquelle ils doivent s'hybrider, mais aussi non strictement complémentaires à la condition qu'ils s'hybrident à la cible. De même, il peut s'agir d'oligonucléotides anti-sens non modifiés ou modifiés au niveau des liaisons inter-nucléotidiques. Toutes ces notions font partie des connaissances générales de l'homme de l'art.

Une composition thérapeutique peut comprendre, entre autres, un vecteur de thérapie génique, la protéine Env HERV-W ou un fragment de cette protéine telle que définie précédemment, et une séquence d'acide nucléique ou oligonucléotide anti-sens tels que définis ci-dessus.

La protéine Env HERV-W ou un de ses fragments est également utilisé comme vecteur thérapeutique pour le transfert d'un gène d'intérêt thérapeutique dans une cellule cible et dans la formulation d'une composition thérapeutique comprenant au moins un vecteur de thérapie génique, la protéine Env HERV-W ou un fragment de cette protéine telle que définie précédemment, et un gène d'intérêt thérapeutique ainsi que les éléments permettant l'expression dudit gène d'intérêt thérapeutique. Les gènes d'intérêt thérapeutique peuvent être non mutés ou mutés. Ils peuvent également consister en des acides nucléiques modifiés de sorte qu'il ne leur est pas possible de s'intégrer dans le génome de la cellule cible ou des acides nucléiques stabilisés à l'aide d'agents, tels que la spermine.

Par éléments assurant l'expression dudit gène d'intérêt thérapeutique *in vivo* on fait notamment référence aux éléments nécessaires pour assurer l'expression dudit gène thérapeutique, après son transfert dans une cellule cible. Il s'agit notamment des séquences promotrices et/ou des séquences de régulation efficaces dans ladite cellule, et éventuellement les séquences requises pour permettre l'expression à la surface des cellules cibles d'un polypeptide. Le promoteur utilisé peut être un promoteur viral, ubiquitaire ou spécifique de tissu ou encore un promoteur synthétique. A titre d'exemple, on mentionnera les promoteurs, tels que les promoteurs des virus RSV (Rous Sarcoma Virus), MPSV, SV40 (Simian Virus), CMV (Cytomegalovirus) ou du virus de la vaccine. Il est en outre possible de choisir une séquence promotrice spécifique d'un type cellulaire donné, ou activable dans des conditions définies. La littérature procure un grand nombre d'informations relatives à de telles séquences promotrices.

Dans un autre mode de réalisation, on peut utiliser dans une composition thérapeutique une cellule exprimant la protéine Env HERV-W ou un fragment de cette protéine tels que définis précédemment comme véhicule de gène(s) de grande taille grâce aux propriétés fusogènes de la protéine ou de ses fragments qui permettent la fusion de la cellule vecteur avec une cellule hôte déficitaire pour un ou des gènes déterminé(s), permettant ainsi de compenser le ou les gène(s) déficitaire(s) (exemple : distrophine).

Les propriétés ou pouvoir fusogène(s) de la protéine ou du polypeptide ci-dessus ou de leurs fragments sont également utilisées dans un procédé pour tester l'efficacité et sélectionner des drogues ou substances médicamenteuses ou des systèmes gène/pro-drogue susceptibles d'intervenir qualitativement et/ou quantitativement sur leur pouvoir fusogène par mise en contact de ladite drogue ou substance médicamenteuse ou dudit système gène/pro-drogue avec des cellules d'une culture cellulaire exprimant ladite protéine ou ledit polypeptide ou ledit fragment et, observation d'une régression ou d'une disparition dans la formation de syncytia, étant entendu que la formation de syncytia à l'état naturel est associée à un état pathologique. A titre d'exemple, on peut citer les phénomènes hémorragiques, la destruction ou l'altération des cellules neuronales, la destruction ou l'altération exacerbée des ostéoblastes.

Une autre application est un procédé pour la sélection de drogues ou substances médicamenteuses ou de systèmes gène/pro-drogue susceptibles d'intervenir qualitativement et/ou quantitativement sur le pouvoir fusogène d'une protéine ou d'un polypeptide ou d'un fragment tel que défini précédemment. Selon ce procédé, on met en contact ladite drogue ou substance médicamenteuse ou ledit système gène/prodrogue avec des cellules d'une culture cellulaire exprimant ladite protéine ou ledit polypeptide ou ledit fragment et, on observe une régression ou une disparition dans la formation de syncytia.

Une autre utilisation d'au moins une séquence d'acide nucléique ou d'au moins un oligonucléotide anti-sens répondant aux critères définis précédemment et susceptible de s'hybrider et d'interférer spécifiquement avec la synthèse de la protéine Env HERV-W et une composition thérapeutique comprenant, entre autres, ladite séquence d'acide nucléique ou oligonucléotide anti-sens vise à obtenir *in vivo* une régression ou une disparition de syncytia associés à un état pathologique.

Dans le but d'obtenir *in vivo* une régression de la formation de syncytia ou une disparition de syncytia associés à un état pathologique, on prépare une composition thérapeutique comprenant, entre autres, un ligand susceptible de reconnaître le récepteur identifié précédemment et d'inactiver ou inhiber le processus de formation de syncytia ou une composition comprenant un gène codant pour un ligand susceptible de s'exprimer *in vivo* dans une cellule cible ou dans un tissu cellulaire cible déterminé, ledit gène étant sous la dépendance des éléments nécessaires assurant son expression,. après son transfert dans la cellule ou le tissu cellulaire cible.

Ainsi, par ligand on entend toute molécule qui est capable de reconnaître ledit récepteur et/ou d'inhiber sa fonction. Il peut s'agir, entre autres, d'un anticorps monoclonal ou d'un anticorps polyclonal ou d'un fragment d'anticorps monoclonal ou d'anticorps polyclonal. Il peut s'agir également d'une molécule inhibitrice de la fonction du récepteur dont la constante d'affinité serait plus grande que celle de la protéine Env HERV-W pour sa liaison et fixation au récepteur.

La production d'anticorps polyclonaux et monoclonaux fait partie des connaissances générales de l'homme du métier. On peut citer à titre de référence Köhler G. et Milstein C. (1975) : Continuous culture of fused cells secreting antibody of predefined specificity, Nature 256 :495-497 et Galfre G. et al. (1977) Nature, 266 : 522-550 pour la production d'anticorps monoclonaux et Roda A., Bolelli G.F. : Production of high-titer antibody to bile acids, Journal of Steroid Biochemistry, Vol. 13, pp 449-454 (1980) pour la production d'anticorps polyclonaux. Pour la production d'anticorps monoclonaux, un immunogène peut être couplé à de l'hémocyanine de Lymphet Keyhole (peptide KLH) comme support pour l'immunisation ou à de l'albumine sérique (peptide SA). Les animaux sont soumis à une injection d'immunogène en utilisant de l'adjuvant complet de Freund. Les sérums et les surnageants de culture d'hybridome issus des animaux immunisés sont analysés pour leur spécificité et leur sélectivité en utilisant des techniques classiques, telles que par exemple des tests ELISA ou de Western Blot. Les hybridomes produisant les anticorps les plus spécifiques et les plus sensibles sont sélectionnés. Des anticorps monoclonaux peuvent également être produits *in vitro* par culture cellulaire des hybridomes produits ou par récupération de liquide d'ascite, après injection intrapéritonéale des hybridomes chez la souris. Quel que soit le mode de production, en surnageant ou en ascite, les anticorps sont ensuite purifiés. Les méthodes de purification utilisées sont essentiellement la filtration sur gel échangeur d'ions et la chromatographie d'exclusion ou l'immunoprécipitation. Un nombre suffisant d'anticorps sont criblés dans des tests fonctionnels pour identifier les anticorps les plus performants. La production *in vitro* d'anticorps, de fragments d'anticorps ou de dérivés d'anticorps, tels que des anticorps chimères produits par génie génétique est bien connue de l'homme du métier.

Plus particulièrement, par fragment d'anticorps on entend les fragments F(ab)2, Fab, Fab', sFv (Blazar et al., 1997, Journal of Immunology 159 : 5821-5833 et Bird et al., 1988, Science 242 : 423-426) d'un anticorps natif et par dérivé on entend, entre autres, un dérivé chimérique d'un anticorps natif (voir par exemple Arakawa et al., 1996, J. Biochem 120 : 657-662 et Chaudray et al., 1989, Nature 339 : 394-397).

Comme évoqué précédemment la thérapie génique ouvre la possibilité d'exprimer *in vivo* de tels ligands par l'administration de compositions thérapeutiques comprenant au moins un gène codant pour un tel ligand. Un tel gène d'intérêt thérapeutique code notamment (i) soit pour au moins un anticorps polyclonal ou monoclonal ou un fragment d'anticorps monoclonal ou polyclonal ou encore pour un anticorps transmembranaire natif, ou un fragment d'un tel anticorps, pour autant que l'anticorps ou fragment d'anticorps soit exprimé *in vivo* à la surface d'une cellule cible ou de cellules cibles d'un tissu et soit capable de reconnaîre et de se lier audit récepteur, (ii) soit pour au moins une molécule inhibitrice comme décrit ci-dessus.

Par cellules cibles ou cellules cibles d'un tissu, telles que définies ci-dessus, on entend (i) soit des cellules au niveau desquelles on veut intervenir pour prévenir ou inhiber la formation de syncytia, (ii) soit des cellules autres mais qui sont susceptibles d'exprimer le ligand et par voie de conséquence d'inhiber et/ou de bloquer l'activité fonctionnelle du récepteur.

Par élément assurant l'expression *in vivo* dudit gène, on fait notamment référence aux éléments nécessaires pour assurer son expression, après transfert dans une cellule cible. Il s'agit notamment des séquences promotrices et/ou des séquences de régulation efficaces dans ladite cellule et éventuellement des séquences requises pour permettre l'expression à leur surface d'un polypeptide ou d'une molécule inhibitrice, tel qu'évoqué ci-dessus. Le promoteur utilisé peut être un promoteur viral, ubiquitaire ou spécifique de tissu ou encore un promoteur synthétique. Des exemples de tels promoteurs ont été décrits précédemment.

Par anticorps transmembranaire, on entend un anticorps dont au moins la région fonctionnelle capable de reconnaître et de se fixer au récepteur est exprimée à la surface des cellules cibles pour permettre la reconnaissance et la fixation. De tels anticorps peuvent consister en des polypeptides de fusion comprenant une séquence d'acides aminés définissant la région fonctionnelle et une séquence d'acides aminés définissant un polypeptide transmembranaire qui permet l'ancrage au sein de la bi-couche lipidique membranaire des cellules cibles ou à la surface externe de cette bi-couche lipidique. Des séquences nucléiques codant pour de tels anticorps transmembranaires sont décrites dans la littéraure.

Par gène ou séquence d'acide nucléique ou leurs fragments, on entend (i) un gène ou un acide nucléique natif isolé ou leurs fragments isolés obtenus par coupure enzymatique, ou (ii) un gène ou acide nucléique ou leurs fragments obtenu(s) par synthèse chimique à l'aide de synthétiseurs automatiques, tels que les synthétiseurs commercialisés par la société Applied Biosystems.

Par cellules tumorales, on entend (i) des cellules de lignées cellulaires immortalisées ou (ii) des cellules primaires tumorales prélevées chez un patient.

Par promoteur autologue, on entend un LTR 5' de HERV-W, à la condition qu'il soit fonctionnel et par promoteur hétérologue on entend tout promoteur n'appartenant pas à la famille de HERV-W, d'origine virale, rétrovirale ou cellulaire, éventuellement modifié, à la condition qu'il soit fonctionnel. Avantageusement, le promoteur autologue ou hétérologue est un promoteur fort c'est-à-dire qu'il est capable d'induire une expression quantitativement importante de la protéine ou du polypeptide.

Le pouvoir fusogène de la protéine Env HERV-W peut également être utilisé pour favoriser le processus d'adhésion cellulaire dans la cas de greffes hétérologues ou homologues ou dans des processus de réparation cellulaire.

### Exemple 1 :

### Lignées cellulaires:

La lignée TELCeB6 (Cosset et al., Journal of Virology, 69 (12) : 7430-7436 (1995)) dérive de la lignée TELac2 après transfection et sélection clonale d'un plasmide d'expression destiné à produire des protéine Gag et Pol de type MoMLV (virus de la leucémie murine de Moloney). La lignée TELac2 dérive initialement des cellules humaines de rhabdomyosarcome TE671 (ATCC CRL 8805) et exprime le vecteur rétroviral rapporteur nlsLacZ (Takeuchi et al., Journal of Virology, 68 (12) : 8001-8007 (1994)). La production de particules rétrovirales infectieuses par les cellules TELCeB6 dépend des vecteurs d'expression d'enveloppe transfectés.

Ces cellules sont cultivées en milieu DMEM (Dulbecco modified Eagle medium - Life Technologies) avec 10% de sérum de veau foetal (Life Technologies). D'une manière générale, ce milieu a été utilisé pour tous les autres types cellulaires, i. e. les cellules TE671 (ATCC CRL 8805 - rhabdomyosarcome humain), A-431 (ATCC CRL-1555 - tumeur solide, carcinome épidermoïde humain), HeLa (ATCC CCL-2), COS (ATCC CRL-1651), PAE (cellules endothéliales d'aorte de porc), XC (ATCC CCL-165 - sarcome de rat), NIH-3T3 et QTB (ATCC CRL-1708).

### Construction des vecteurs d'expression d'enveloppe :

Le plasmide pHCMV a été utilisé pour l'expression de *env* HERV-W. Le plasmide FBASALF-ARless a été utilisé en qualité de témoin positif de fusion; il produit une forme hautement fusogénique de la glycoprotéine d'enveloppe MLV amphotrope, modifiée par introduction d'un codon stop avant le premier acide aminé du peptide intracytoplasmique p2-R (Rein et al., Journal of Virology, 68 (3) : 1773-1781 (1994)). *env* HERV-W cloné en anti-sens dans le plasmide pHCMV a été utilisé comme témoin négatif.

### Transfection et tests de fusion de cellule à cellule (coculture) :

Les plasmides d'expression des glycoprotéines d'enveloppe sont transfectés dans les cellules TELCeB6 par précipitation au phosphate de calcium (Cosset et al., Journal of Virology, 69 (10) : 6314-6322 (1995)). Les cellules TELCeB6 confluentes exprimant Env sont fixées au Glutaraldéhyde à 0,5% en PBS, 24 h. après transfection. Une coloration par des solutions de May-Grünwald et Giemsa (MERCK) est alors effectuée selon les recommandations du fournisseur. Elle colore les noyaux en violet et les cytoplasmes en mauve et permet de visualiser les syncytia.

Pour les expériences de coculture, les cellules transfectées sont décrochées du support, comptées puis ré-ensemencées à concentration égale (3 x 10⁵ cellules/puit) en plaques 6 puits. Des cellules fraîches indicatrices sont alors ajoutées aux cellules transfectées à raison de 10⁶ cellules par puit et la co-culture est poursuivie pendant 24 h. Une coloration XGal (5-bromo-4chloro-3-indolyl-β-D-galactopyranoside peut alors être effectuée pour colorer le noyau des cellules TELCeB6 (Cosset et al., Journal of Virology, 69 (10) : 6314-6322 (1995)). Elle est suivie d'une coloration par des solutions de May-Grünwald et Giemsa (MERCK) effectuée selon les recommandations du fournisseur.

La majorité des syncytia est observable 18 à 24 heures après le début de la transfection ; le décollement progressif des cellules ne permet plus d'observation, ni de coloration 36 heures après la transfection. La fusion observée correspond à une fusion " from within ", c'est-à-dire à une fusion de cellule-à-cellule, à partir d'une cellule exprimant l'enveloppe, par opposition à une fusion " from without " qui correspond à une formation de syncytia consécutive à une fusion virion-cellule(s).

Le tableau I ci-après rassemble les résultats obtenus concernant la capacité de fusion de cellule à cellule de Env HERV-W par transfection directe, comparée à celle de l'enveloppe témoin ARless. Les cellules TELCeB6 et TE671 correspondent à des lignées d'origine humaine. Les cellules COS sont des cellules de rein de singe vert. Les cellules XC sont des cellules de rat.

**Tableau I**

| | index^{a} de fusion sur les cellules | | | |
|---|---|---|---|---|
| **Enveloppe** | **TELCeB6** | **TE671** | **COS** | **XC** |
| ARless | 33 | 8,6 | inn. | 40 |
| HERV-W | 61 | 24,7 | 3,6 | 0 |

| | | | | |
|---|---|---|---|---|
| ^{a}L'index de fusion correspond au pourcentage *(N-S)*/*T*, où *N* est le nombre de noyaux en syncytia, *S* est le nombre de syncytia et *T* est le nombre total de noyaux comptés. inn. signifie innombrables, organisés en "réseau". | | | | |

Le tableau I montre que les résultats sont au moins aussi importants pour Env HERV-W que pour le témoin sur les cellules d'origine humaine. Ils sont moindres sur les cellules simiennes. Env HERV-W n'induit pas la formation de syncytia sur des cellules de rat.

Le tableau II ci-après rassemble les données observées dans des expériences de coculture de cellules indicatrices avec des cellules TELCeB6 transfectées par pHCMV-*env* HERV-W. Le type, l'origine et l'espèce des cellules indicatrices sont indiqués. La formation de syncytia est indiquée par les mentions oui/non.

**Tableau II**

| Espèce | Type cellulaire | Origine | Fusion en co-culture avec TELCeB6 |
|---|---|---|---|
| Homme | TE671 | Rhabdomyosarcome | Oui |
| | A431 | Carcinome épidermoïde | Oui |
| | HeLa | Carcinome épithélioïde | Oui |
| Singe | COS | Type fibroblastique | Oui |
| Porc | PAE | Endothélium | Oui |
| Rat | XC | Sarcome | Non |
| Souris | 3T3 | Fibroblastique | Non |
| Caille | QT6 | Fibrosarcome | Non |

Le tableau II détaille les résultats des expériences de coculture en fonction des lignées cellulaires testées. On observe des syncytia dans des cellules humaines de rhabdomyosarcome (TE671), de carcinome épidermoïde (A431) et de carcinome épithélioïde (HeLa), ainsi que dans des cellules de singe de type fibroblastique (COS), des cellules de porc d'endothélium (PAE) et des cellules de souris de type fibroblastique (3T3). Le fait que l'enveloppe endogène humaine Env HERV-W soit capable de fusionner dans des cellules de porc peut poser des problèmes dans le cadre de la transplantation d'organes (xénotransplantation).

### Exemple 2 :

### Amplification conjointe puis sélective de la LTR et de l'enveloppe:

Afin d'étudier le polymorphisme de la région codante de l'enveloppe et de la région promotrice U3 de la LTR 5' associée, situées sur le chromosome 7, une amplification spécifique d'un fragment de 10kb est réalisée grâce à un couple d'amorces spécifiques. En effet, sachant que la famille HERV-W comporte de nombreuses copies non codantes et en particulier un nombre important de LTR, cette stratégie permet d'amplifier spécifiquement et conjointement la région *env* et sa séquence promotrice (LTR5') située en amont, exclusivement sur le chromosome 7. On utilise pour cela une amorce U6198 s'hybridant sur une séquence spécifique située en amont de la LTR 5' sur le chromosome 7, et une amorce L6186 s'hybridant de façon chevauchante sur la région U5 du LTR 3' et le gène cellulaire adjacent, sur ce même chromosome. La longue PCR (ou LD-PCR) est réalisée dans les conditions suivantes, 1 x 5 min. à 94°C, 10 x (10 sec à 94°C, 30 sec à 55°C, 8 min. à 68°C), 25 x (10 sec à 94°C, 30 sec à 55°C, 8 min. à 68°C + 10 sec /cycle), 1 x 7 min. à 68°C, en présence de tampon d'amplification (50 mM Tris HCL pH 9,0 à 25°C, 15mM (NH₄)₂SO₄, 0.1% Triton X-100); 1,5 mM MgCl₂, 0,25mM de chaque dNTP, 330 nM de chaque amorce (U6198 et L6186), 1U d'ADN polymérase ainsi que 200ng de matrice (ADN génomique) dans un volume final de 50ml.

A partir de ce produit PCR de 10kb dilué, une PCR nichée "env" ainsi qu'une PCR nichée "LTR" sont effectuées, afin d'objectiver la présence ou non d'un polymorphisme de ces deux régions. La dilution permet une amplification spécifique à partir du produit de LD-PCR et non du matériel génomique de départ. La PCR nichée "env" est réalisée grâce aux amorces U6189 et L6186, l'amorce U6189 étant celle employée pour la LD-PCR, l'amorce U6189 étant située en amont de l'ATG de env. La région U3 du LTR 5' est amplifiée par le couple d'amorce U6460 et L5643. L'amorce U6460 s'hybride en amont de la LTR 5' tandis que l'amorce L5643 s'hybride dans le domaine R de la LTR 5'. Les PCR nichées sont réalisées dans les conditions suivantes, 1 x 5 min. à 94°C, 30 x (1 min. à 94°C, 1 min. à 53°C, 3 min. à 72°C), 1 x 7 min. à 72°C, en présence de tampon d'amplification (10 mM Tris HCL pH 8,3, 50mM KCl), 1,5 mM MgCl₂, 0,25mM de chaque dNTP, 330 nM de chaque amorce, 1,25U d'ADN polymérase, un aliquot du produit d'amplification de la LD-PCR, dans un volume final de 50ml.

### Analyse du polymorphisme:

Afin d'objectiver la présence ou non d'un polymorphisme, les produits des PCR nichés peuvent être analysés de différentes façons, en particulier le séquençage ou l'analyse par la technique SSCP (Single Strand Conformation Polymorphism) permettant de mettre en évidence la présence d'au moins une mutation entre deux séquences courtes d'une taille moyenne de 250pb.

Polymorphisme du gène *env*: l'utilisation de 20 amorces (10 amorces sens paires: 6302 à 6320 et 10 amorces anti-sens impaires: 6303 à 6321) permet le séquençage de la région codante de l'enveloppe à partir du produit PCR nichée enveloppe. Ces amorces peuvent également être utilisées pour une analyse du polymorphisme par SSCP. A titre d'exemple, les séquences des gènes d'enveloppe de trois donneurs sains étiquetés D6, D10 et D21 sont illustrées à la figure 2. Ces séquences montrent l'existence d'un faible taux de polymorphisme. Si on utilise la séquence d'enveloppe du donneur D6 comme référence arbitraire, la séquence de l'enveloppe du donneur D21 possède une mutation en position 386 (T386C), le remplacement de la thymine par la cytosine induisant un changement d'acide aminé de valine en alanine (V128A en numérotation protéique). De même, la séquence du gène de l'enveloppe du donneur D10 présente deux mutations par rapport à la séquence du donneur D6, en position 671 (T671C) et 920 (G920A), induisant deux changements d'acides aminés, respectivement de valine en alanine (V224A en numérotation protéique) et de sérine en asparagine (S306N en numérotation protéique). Ces séquences illustrent l'existence d'un polymorphisme Le séquençage de 12 ADN de patients a été réalisé et nous a permis d'observer un faible taux de polymorphisme entre les ADN testés. Par exemple, la comparaison des séquences issues de deux individus notés 10 et 21 montre la présence de trois bases de différence en acides nucléiques sur les 1617 bases du gène, ce qui correspond à un taux de polymorphisme de 0,19%. Deux mutations sont situées sur la séquence de l'ADN 10 (T671C et G920A) et une sur la séquence de l'ADN 21 (T386C). La séquence de l'individu 6 sert de référence. Cette même analyse au niveau protéique permet d'observer 3 acides aminés mutés pour l'enveloppe entière comportant au total 538 acides aminés, soit un taux de polymorphisme de 0,56%. Les deux mutations de la séquence issue de l'individu 10 sont V224A et S306N et celle de la séquence issue de l'individu 21 est V128A.

Polymorphisme de la région promotrice U3 de la LTR5' associée au gène d'enveloppe: le séquençage du domaine U3 du LTR 5' est réalisé grâce aux 2 amorces précédemment utilisées pour la PCR nichée LTR. A titre d'exemple, les séquences de la région U3 de la LTR5' (associée au gène de l'enveloppe) de deux des donneurs sains (étiquetés D6 et D21) pour lesquels l'enveloppe a par ailleurs été séquencée sont illustrées à la figure 3. Ces séquences montrent l'existence d'un taux de polymorphisme plus important que pour le gène de l'enveloppe. On notera en particulier les variations aux positions 210 (T pour D6, C pour D21), 211 (G pour D6, A pour D21), 229 (A pour D6, G pour D21), 231 (T pour D6, C pour D21), 232 (C pour D6, A pour D21)

Les séquences des amorces utilisées pour la PCR, le SSCP et le séquençage sont illustrés dans le tableau III ci-dessous.

**Tableau III**

| **NOM:** | **SEQUENCES NUCLEOTIDIQUES:** |
|---|---|
| | Amorces PCR longues |
| U6198 : SEQ ID NO :3 | 5'- CAA-AAC-GCC-TGG-AGA-TAC-AGC-AAT-TAT-C-3' |
| L6186 : SEQ ID NO:4 | 5'- GCA-CCC-TCA-TGG-TTG-TGT-TAC-TTG-G-3' |

| | Amorces PCR nichée *env* |
|---|---|
| U6189 : SEQ ID NO:5 | 5'- CTG-AAA-ATC-CAG-GAG-ACA-ACG-CTA-GC-3' |
| L6186 : SEQ ID NO:6 | 5'- GCA-CCC-TCA-TGG-TTG-TGT-TAC-TTG-G-3' |

| | Amorces PCR nichée LTR 5' |
|---|---|
| U6460 : SEQ ID NO :7 | 5'-TTG-GTA-CCC-AAA-ACG-CCT-GGA-GAT-ACA-GCA-ATT-ATC-3' |
| L5643 : SEQ ID NO:8 | 5'- AAC-TCG-AGT-GAA-ATA-GCA-TGA-AAA-CAG-AG-3' |

| | Amorces SSCP et séquençage *env*: |
|---|---|
| U6302 : SEQ ID NO :9 | 5'- AGG-AAA-GTA-ACT-AAA-ATC-ATA-AAT-C-3' |
| L6303 : SEQ ID NO : 10 | 5'- GGT-TCC-CTT-AGA-AAG-ACT-CC-3' |
| U6304 : SEQ ID NO :11 | 5'- AAT-ATT-GAT-GCC-CCA-TCG-TAT-A-3' |
| L6305 : SEQ ID NO :12 | 5'- CCA-GTT-TGG-GTG-AAG-TAA-GTC-3' |
| U6306 : SEQ ID NO :13 | 5'- GGA-GGA-CTT-GGA-GTC-ACT-GTC-3' |
| L6307 : SEQ ID NO :14 | 5'- AGG-CGA-GTA-TGG-GTA-CGG-AG-3' |
| U6308 : SEQ ID NO :15 | 5'- GGA-CTA-GAT-CTC-TCA-AAA-CTA-CA-3' |
| L6309 : SEQ ID NO :16 | 5'- ACG-GAA-GTG-GTG-TTT-ATT-TCT-G-3' |
| U6310 : SEQ ID NO :17 | 5'- CCT-GAA-CAA-TGG-AAC-AAC-TTC-3' |
| L6311 : SEQ ID NO :18 | 5'- ATT-CCT-GAG-GGT-AGG-CAG-AC-3' |
| U6312 : SEQ ID NO :19 | 5'- GGT-AAC-TCC-TCC-CAC-ACA-AA-3' |
| L6313 : SEQ ID NO :20 | 5'- GAA-TGG-GTA-CTC-TTT-TGT-TGC-3' |
| U6314 : SEQ ID NO :21 | 5'- TAC-AGT-TAT-GTC-ATA-TCT-AAG-CC-3' |
| L6315 : SEQ ID NO :22 | 5'- TAA-GTT-GAT-CTT-GCA-AGG-TGA-C-3' |
| U6316 : SEQ ID NO :23 | 5'- CTA-AAT-GGG-GAC-ATG-GAA-CG-3' |
| L6317 : SEQ ID NO :24 | 5'- TAT-TCG-ATC-TGG-AAT-TTC-TTC-AAC-3' |
| U6318 : SEQ ID NO :25 | 5'- CAA-TCC-GGA-ATC-GTC-ACT-GA-3' |
| L6319 : SEQ ID NO :26 | 5'- AGA-CAA-AGT-TAA-CAA-GGA-GGT-TC-3' |
| U6320 : SEQ ID NO :27 | 5'- ACT-CCT-CTT-TGG-ACC-CTG-TAT-C-3' |
| L6321 : SEQ ID NO :28 | 5'-GAG-GTT-GGC-CGA-CCA-CCG-3' |

| | |
|---|---|
| U fait référence à des amorces sens et L fait référence à des amorces réverses. | |

### Exemple 3 :

Afin de déterminer le récepteur reconnu par la glycoprotéine d'enveloppe de HERV-W parmi les récepteurs connus comme étant exprimés dans les cellules humaines, c'est à dire PiT-2 (le récepteur des MLV amphotropes), PiT-1 (le récepteur des GALV -gibbon ape leukemia virus et FeLV-B -feline leukemia virus type B) et hATB° (le récepteur des rétrovirus mammifères de type D également reconnu par le rétrovirus RD114), des tests d'interférence ont été réalisés. Pour cela, des cellules TELCeB6 ont été transfectées, soit avec le plasmide d'expression codant pour l'enveloppe HERV-W, soit avec le plasmide d'expression exprimant l'ARN messager anti-sens du gène codant pour l'enveloppe HERV-W, soit avec le plasmide d'expression codant pour un variant hyperfusogénique de l'enveloppe MLV amphotrope nommé ARless. Ces cellules, appelées "cellules productrices" ont ensuite été co-cultivées avec des cellules humaines, dites "cellules indicatrices", exprimant le récepteur de l'enveloppe HERV-W, et qui expriment, de plus, de manière stable, soit l'enveloppe du GALV, soit l'enveloppe du MLV amphotrope, soit l'enveloppe du RD114. L'expression des ces diverses glycoprotéines d'enveloppe sur ces cellules est capable de reconnaître les récepteurs correspondants, de les bloquer et donc de diminuer leur capacité à interagir avec une glycoprotéine d'enveloppe rétrovirale correspondante, mais exprimée de manière exogène, à la surface des cellules "productrices". Ainsi, si lors des tests de fusion par co-culture, on observe une diminution dans la formation de syncytia pour un type cellulaire indicateur bloquant un de ces récepteurs par comparaison à la cellule indicatrice parentale pour laquelle l'ensemble des trois récepteurs potentiels est pleinement accessible, on pourra en déduire la nature du récepteur reconnu par l'enveloppe exprimée sur la cellule productrice. Après deux jours de co-culture, les cellules ont été fixées, colorées, et les indices de fusion déterminés. Les résultats sont présentés dans le tableau IV ci-dessous.

**Tableau IV**

| Protéine d'enveloppe exprimée dans les cellules productrices. | Protéines d'enveloppe exprimées dans les cellules indicatrices. | | | |
|---|---|---|---|---|
| | Témoin | MLV-A | GALV | RD114 |
| ARless | + | - | + | + |
| anti-sens HERV-W | - | - | - | - |
| HERV-W | + | + | + | - |

| | | | | |
|---|---|---|---|---|
| - signifie une absence de syncytia et + signifie la présence de syncytia Témoin signifie qu'il n'y a pas de protéine d'enveloppe exprimée dans cette cellule. | | | | |

Ces résultats permettent de déduire que la glycoprotéine d'enveloppe de HERV-W reconnaît le récepteur hATB° des rétrovirus mammifères de type D. En effet, alors que cette enveloppe est fusogénique pour les cellules indicatrices parentales ou pour les cellules indicatrices exprimant, soit l'enveloppe MLV-A, soit l'enveloppe GALV, on n'observe pas de syncytia quand les cellules productrices exprimant la glycoprotéine d'enveloppe de HERV-W sont co-cultivées avec les cellules indicatrices exprimant l'enveloppe RD114.

### Exemple 4 : Contrôle de l'activité fusogène de HERV-W Env par sa partie cytoplasmique.

L'implication de la partie cytoplasmique de HERV-W Env dans l'activité fusogène de cette glycoprotéine est démontrée par la construction et la caractérisation des glycoprotéines recombinantes suivantes :

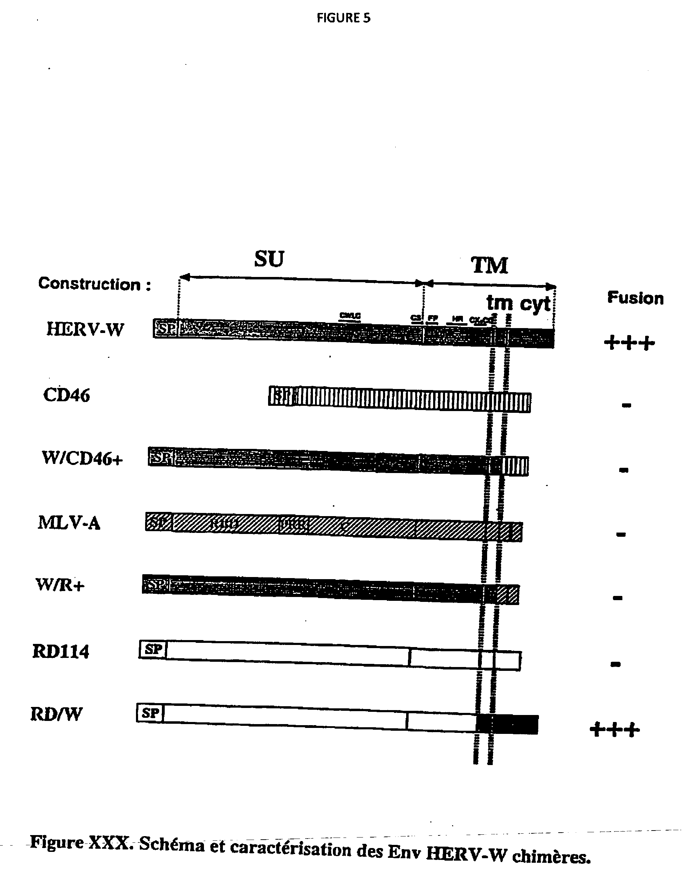

W/CD46+, dérivé du CD46 humain, facteur de protection des cellules vis-à-vis du complément et non impliqué dans la formation de syncytia, comportant l'ectodomaine et le domaine transmembranaire de HERV-W Env (aa 1 à 469) fusionnés au domaine cytoplasmique du CD46 (aa 335 à 369). Cette molécule chimère n'est pas fusogénique dans un test de fusion cellule-cellule (Figure 5).

W/R+, dérivé de la glycoprotéine d'enveloppe du rétrovirus MLV-A (amphotropic murine leukemia virus), non fusogénique quand exprimée indépendamment des autres protéines de MLV-A, comportant l'ectodomaine et le domaine transmembranaire de HERV-W Env (aa 1 à 469) fusionnés au domaine cytoplasmique de la glycoprotéine d'enveloppe du rétrovirus MLV-A (aa 622 à 654). Cette molécule chimère n'est pas fusogénique dans un test de fusion cellule-cellule (Figure 5).

RD/W, dérivé de la glycoprotéine d'enveloppe du rétrovirus endogène félin RD114, non fusogénique quand exprimée indépendamment des autres protéines du RD114, comportant le domaine cytoplasmique et le domaine transmembranaire de HERV-W Env (aa 448 à 538) fusionnés à l'ectodomaine de la glycoprotéine d'enveloppe du rétrovirus RD114 (aa 1 à 508). Cette molécule chimère est fusogénique dans un test de fusion cellule-cellule (Figure 5).

La figure 5 annexée représente le schéma et la caractérisation des chimères Env HERV-W précitées et les résultats obtenus dans un test de fusion cellule-cellule.

L'ectodomaine de HERV-W Env est défini par le polypeptide dérivé du précurseur protéique contenant les acides-aminés (aa) 21 à 447; le domaine transmembranaire, les aa 448 à 469 et la partie cytoplasmique, les aa 470 à 538 (Blond et al., (1999) Molecular characterization and placentalexpression of HERV-W, a new human endogenous retrovirus family. Journal of Virology. 73:1175-1185).

L'ectodomaine de CD46 est défini par le polypeptide dérivé du précurseur protéique contenant les acides-aminés (aa) 35 à 312; le domaine transmembranaire, les aa 313 à 334 et la partie cytoplasmique, les aa 335 à 369 (Yant et al., (1997), Identification of a cytoplasmic Tyr-X-X-Leu motif essential for down regulation of the human cell receptor CD46 in persistent measles virus infection. J Virol.71:766-770).

L'ectodomaine de MLV-A Env est défini par le polypeptide dérivé du précurseur protéique contenant les acides-aminés (aa) 32 à 598; le domaine transmembranaire, les aa 599 à 621 et la partie cytoplasmique, les aa 622 à 654 (Ott et Rein, (1990), Sequence analysis of amphotropic and 10A1 murine leukemia virus: close relationship to mink cell focus forming viruses. J Virol. 64:757-766).

L'ectodomaine de RD114 Env est défini par le polypeptide dérivé du précurseur protéique contenant les acides-aminés (aa) 18 à 508; le domaine transmembranaire, les aa 509 à 530 et la partie cytoplasmique, les aa 531 à 564 (Cosset et al., (1995b), High titer packaging cells producing recombinant retroviruses resistant to human serum. J. Virol. 69:7430-7436).

SU signifie sous-unité de surface; TM sous-unité transmembranaire; SP peptide signal; tm domaine d'ancrage transmembranaire; cyt partie cytoplasmique; RBD domaine de liaison au récepteur; PRR région riche en proline; C domaine carboxy-terminal de la SU.

## Revendications

1. Utilisation d'un fragment d'acide nucléique codant pour l'expression d'un peptide d'une protéine d'enveloppe du rétrovirus endogène humain HERV-W, ledit peptide commençant à l'acide aminé 448 et se terminant à l'acide aminé 538 de SEQ ID NO : 1, pour préparer une composition de thérapie génique, destinée au traitement de cancers par destruction des cellules indicatrices cancéreuses exprimant le récepteur hATB°, au moyen de la formation de syncytia.

2. Utilisation d'un gène codant pour l'expression d'une protéine d'enveloppe du rétrovirus endogène humain HERV-W, ladite protéine comprenant ou consistant en SEQ ID NO: 1, pour préparer une composition de thérapie génique, destinée au traitement de cancers par destruction des cellules indicatrices cancéreuses exprimant le récepteur hATB°, au moyen de la formation de syncytia.

3. Utilisation selon la revendication 1 ou 2, **caractérisée en ce que** la composition comprend un promoteur hétérologue ou autologue, de préférence hétérologue, pour l'expression dudit peptide ou de ladite protéine.

4. Utilisation selon l'une quelconque des revendications 1 à 3, **caractérisée en ce que** la composition comprend un vecteur de thérapie génique comprenant ledit peptide ou ladite protéine.

5. Utilisation selon la revendication 4, **caractérisée en ce que** le vecteur est choisi parmi un vecteur rétroviral de type MLV, un vecteur lentiviral pseudotypés par ladite protéine, un vecteur synthétique.

6. Utilisation selon l'une quelconque des revendications 1 à 5, **caractérisée en ce que** les cellules cancéreuses sont détruites par formation de syncytia entre des cellules transformées par ledit vecteur et lesdites cellules cancéreuses.

7. Utilisation selon l'une quelconque des revendications 1 à 5, **caractérisée en ce que** les cellules cancéreuses sont transformées par ledit vecteur.

## Patentansprüche

1. Verwendung eines für die Expression eines Peptids eines Hüllproteins des humanen endogenen Retrovirus HERV-W kodierenden Nukleinsäureproteins, wobei das Peptid mit der Aminosäure 448 beginnt und mit der Aminosäure 538 der SEQ ID NR. 1 endet, zur Herstellung einer gentherapeutischen Zusammensetzung, die zur Behandlung von Krebs durch Zerstörung der Krebsindikatorzellen, die den Rezeptor hATB° exprimieren, mittels der Ausbildung von Synzytien bestimmt sind.

2. Verwendung eines Gens, das für die Expression eines Hüllproteins des humanen endogenen Retrovirus HERV-W kodiert, wobei das Protein SEQ ID NR. 1 umfasst oder daraus besteht, zur Herstellung einer gentherapeutischen Zusammensetzung, die zur Behandlung von Krebs durch Zerstörung der Krebsindikatorzellen, die den Rezeptor hATB° exprimieren, mittels der Ausbildung von Synzytien bestimmt sind.

3. Verwendung nach Anspruch 1 oder 2, **dadurch gekennzeichnet, dass** die Zusammensetzung einen heterologen oder autologen, vorzugsweise heterologen, Promoter, für die Expression des Peptids oder des Proteins umfasst.

4. Verwendung nach einem der Ansprüche 1 bis 3, **dadurch gekennzeichnet, dass** die Zusammensetzung einen gentherapeutischen Vektor umfasst, der das Peptid oder das Protein umfasst.

5. Verwendung nach Anspruch 4, **dadurch gekennzeichnet, dass** der Vektor aus einem retroviralen Vektor des Typs MLV, einem lentiviralen, durch das Protein pseudotypisierten Vektor, einem synthetischen Vektor ausgewählt ist.

6. Verwendung nach einem der Ansprüche 1 bis 5, **dadurch gekennzeichnet, dass** die Krebszellen durch die Ausbildung von Synzytien zwischen von dem Vektor transformierten Zellen und den Krebszellen zerstört werden.

7. Verwendung nach einem der Ansprüche 1 bis 5, **dadurch gekennzeichnet, dass** die Krebszellen von dem Vektor transformiert werden.

## Claims

1. The use of a nucleic acid fragment coding for the expression of a peptide of an envelope protein of the human endogenous retrovirus HERV-W, said peptide beginning at the amino acid 448 and ending with the amino acid 538 of SEQ ID NO: 1, for preparing a gene therapy composition intended for the treatment of cancers by destruction of cancer indicator cells expressing the receptor hATB°, by means of syncytia formation.

2. The use of a gene coding for the expression of an envelope protein of the human endogenous retrovirus HERV-W, said protein comprising or consisting in SEQ ID NO: 1, for preparing a gene therapy composition, intended for the treatment of cancers by destruction of cancer-indicating cells expressing the receptor hATB°, by means of syncytia formation.

3. The use according to claim 1 or 2, **characterized in that** the composition comprises a heterologous or autologous promoter, preferably a heterologous promoter, for expressing said peptide or said protein.

4. The use according to any of claims 1 to 3, **characterized in that** the composition comprises a gene therapy vector comprising said peptide or said protein.

5. The use according to claim 4, **characterized in that** the vector is selected from a retroviral vector of the MLV type, a lentiviral vector pseudo-typed by said protein, a synthetic vector.

6. The use according to any of claims 1 to 5, **characterized in that** the cancer cells are destroyed by formation of syncytia between cells transformed by said vector and said cancer cells.

7. The use according to any of claims 1 to 5, **characterized in that** the cancer cells are transformed by said vector.
